# EUROPÄISCHE PATENTSCHRIFT

(11) **EP 2 359 899 B1**
(45) Veröffentlichungstag und Bekanntmachung des Hinweises auf die Patenterteilung: **17.06.2015**
(21) Anmeldenummer: 11153008.5
(22) Anmeldetag: 02.02.2011
(51) Int. Cl.: A61N 1/375, A61N 1/08, A61N 1/05, G01R 33/28, H03H 1/00, H03H 7/01

(54) **Elektrodenvorrichtung für aktive medizinische Implantate**
Electrode device for active medical implants
Dispositif d'électrodes pour implants médicaux actifs

(30) Priorität: 11.02.2010 DE 102010000372
(43) Veröffentlichungstag der Anmeldung: 24.08.2011
(73) Patentinhaber: BIOTRONIK SE & Co. KG, 12359 Berlin (DE)
(72) Erfinder: Weiss, Ingo, 12435, Berlin (DE); Friedrich, Michael, 14532, Kleinmachnow (DE); Knorr, Stefan, 10119, Berlin (DE); Fischer, René, 10247, Berlin (DE); Schurr, Marc Steffen, 10179, Berlin (DE)
(74) Vertreter: Lindner-Vogt, Karin L.

(56) Entgegenhaltungen:
- US-A1- 2004 176 817
- US-A1- 2007 217 121
- US-A1- 2009 243 756
- US-B1- 6 567 703

## Beschreibung

Die Erfindung betrifft eine Elektrodenvorrichtung für aktive medizinische Implantate, die einen langgestreckten Elektrodenkörper mit einem proximalen und einem distalen Ende, einen Spitzen-Kontaktpol am distalen Ende und/oder einen Ring-Kontaktpol vor dem distalen Ende, elektrische Zuleitungen zu dem Spitzen- bzw. Ring-Kontaktpol und einen aus einem oder mehreren elektronischen Bauelementen bestehenden Hochfrequenz-Filter in mindestens einer der Zuleitungen umfasst, der dem Spitzen- bzw. Ring-Kontaktpol zugeordnet ist.

Zum Hintergrund der Erfindung ist festzuhalten, dass der Erfindungsgegenstand in erster Linie im Zusammenhang mit Herzschrittmachern, implantierbaren Defibrillatoren und anderen Typen von aktiven implantierbaren elektromedizinischen Geräten relevant ist. Letztere weisen in aller Regel mindestens eine strom-/spannungsführende Zuleitung in der Elektrodenvorrichtung - üblicherweise als kurz als "Elektrode" bezeichnet - auf, deren distales Ende beispielsweise in einer Herzkammer angeordnet und zur Messung kardiologischer Potentialsignale oder zur Abgabe entsprechender therapeutischer Stromsignale dient.

Die Kompatibilität derartiger Elektrodenvorrichtungen bei implantierbaren elektromedizinischen Geräten mit hochfrequenten Magnetfeldern, wie sie insbesondere bei bildgebenden diagnostischen Verfahren auf Magnetresonanz-Basis - sogenannte MRI- (magnetic resonance imaging) Verfahren - verwendet werden, stellt ein gravierendes Problem dar. Bei solchen MRI-Verfahren wird einem starken statischen Magnetfeld ein mit Radiofrequenz (RF) gepulstes magnetisches Wechselfeld überlagert, das dazu dient, den Energiestatus der Protonen im untersuchten Gewebe zu verändern und entsprechende MRI-Signale aus dem Gewebe zu produzieren.

Dieses magnetische Wechselfeld führt nach den Gesetzen der elektromagnetischen Induktion in den Zuleitung der hier in Rede stehenden Elektrodenvorrichtungen von elektromedizinischen Geräteimplantaten zu Wechselspannungen, deren Energie insbesondere an den elektrisch leitenden Kontaktpolen der Elektrodenvorrichtung zum menschlichen Gewebe in Wärme umgesetzt wird. Dies kann zu einer erheblichen Erhitzung beispielsweise des Spitzenkontaktes einer Herzelektrode mit einer entsprechenden Beeinträchtigung und sogar Schädigung des damit in Berührung stehenden oder umliegenden Herzgewebes führen.

Um diese Problematik zu vermeiden, schlägt die US 7,363,090 B2 die Verwendung von Filtern auf der Basis von Schwingkreisen aus parallel geschalteter Spule und Kondensator vor, die der entsprechenden Zuleitung für den Spitzen-Kontaktpol oder einen Ring-Kontaktpol einer entsprechenden Elektrode eines implantierbaren elektromedizinischen Gerätes zugeordnet ist. Die in diesem vorbekannten Patent offenbarten Filter sind in der Praxisumsetzung der Patentinhaberin als vergleichsweise voluminöse, die Elektrodenvorrichtung auf einer gewissen Länge versteifende Komponenten ausgebildet, die der damit ausgerüsteten Elektrode ungünstige mechanische Eigenschaften verleihen. Ferner ist der Filter in einem in sich geschlossenen Gehäuse untergebracht, das keine Durchführung für die in aller Regel beim Implantieren einer Elektrode verwendeten Führungsdrähte bereit stellt. Insoweit sind die Einsatzmöglichkeiten dieser bekannten Elektrode mit Filtereinrichtung begrenzt.

Aus der US 2009/0281592 A1 sind Filterkomponenten zur Herabsetzung der Erhitzung von Schrittmacher-Elektroden eines elektromedizinischen Implantats bei der Einwirkung hochfrequenter Magnetfelder in Folge von MRI-Prozeduren bekannt, wobei eine Induktionsspule um einen nicht-leitenden, zentralen Abschnitt eines Schaftes aufgebracht ist, der einen Spitzen-Kontaktpol mit einem inneren Wendelleiter der Elektrodenvorrichtung verbindet. Durch die Montage einer Induktionsspule auf dem Schaft wird eine induktive Signalfilterung zur Herabsetzung der Elektrodenspitze ohne die Notwendigkeit eines vergleichsweise langen, voluminösen Spulenkörpers entlang der Länge der Elektrode erreicht. Kapazitive Elemente können ebenfalls in den Schaft integriert werden, um einen LC-Filterkreis zu schaffen. Alternativ dazu ist in dieser Druckschrift eine sogenannte "Luftspule" als induktives Element offenbart, bei der der Schaft weggelassen werden kann.

Die Filtereinrichtungen nach dem Stand der Technik führen in der Regel zu einer übermäßigen Versteifung der Elektrodenvorrichtung auf einer bestimmten Länge. Sie sind vergleichsweise aufwendig und wenig kompakt ausgebildet

Eine Elektrodenvorrichtung gemäß Oberbegriff des Anspruchs 1 ist aus US2009/0243756 A1 bekannt. Im Zusammenhang mit schmalbandigen HF-Filtern wird dort auf die Schrift US2007/0112398 A1 verwiesen, die sandwichartige Spulen aus wechselweise übereinander geschichteten Metalsprialen und Keramiklagen beschreibt. Benachbarte Spiralen sind dabei an ihren offenen Enden über Durchführungen elektrisch verbunden.

Davon ausgehend liegt der Erfindung die Aufgabe zugrunde, alternative Ausführungen für Spulen in einem Hochfrequenz-Filter einer Elektrodenvorrichtung für medizinische Implantate aufzuzeigen

Diese Aufgabe wird durch die im Kennzeichnungsteil des Patentanspruchs 1 angegebenen Merkmale gelöst.

Solche LTCC-Bauelemente sind aus Niedertemperatur-Einbrand-Keramiken (Low Temperature Cofired Ceramics) hergestellt und beruhen auf einer Technologie zur Herstellung von Mehrlagenschaltungen auf der Basis von gesinterten Keramikträgern. Damit können grundsätzlich Leiterbahnen, Kondensatoren, Widerstände und Spulen erzeugt werden, die durch Siebdruck oder fotochemische Prozesse aufgebracht sind. Die ungebrannten Keramikfolien werden einzeln strukturiert, danach gestapelt und laminiert. Abschließend wird ein definiertes Sinterprofil mit einer Spitzentemperatur von ca. 850 bis 900°C gefahren. Diese Technologie vereint die Vorteile der Hochtemperatur-Einbrand-Keramik-Technik und der Dickschichttechnologie. Sie ist vor allem bei kleinen und mittleren Stückzahlen eine kostengünstige Alternative zur herkömmlichen Leiterplattentechnologie, was sich günstig im Bereich der Medizintechnik auswirkt.

Mithilfe solcher LTCC-Bauelemente kann ein hoher Integrationsgrad der damit hergestellten Hochfrequenz-Filter erzielt werden, was sich äußerst günstig im Hinblick auf deren geringes Bauvolumen auswirkt. Durch die kleinbauenden Hochfrequenz-Filter führen diese dann zu einer geringeren Versteifung der Elektrodenvorrichtung im Vergleich zur herkömmlichen Realisierung dieser Filtereinrichtungen.

Gemäß einer bevorzugten Ausführungsform kann der Hochfrequenz-Filter einen Kondensator und eine Spule in LTCC-Technik aufweisen, wobei ein Kondensator als LTCC-Bauteil in ein dielektrisches Material integriert sein kann. Eine Montage eines Kondensators als LTCC-Bauteil auf einem dielektrischen Material durch Löten, Schweißen oder Kleben mit leitfähigem Klebstoff ist ebenfalls möglich.

Sofern als dielektrisches Material ein keramisches Dielektrikum vorgesehen ist, kann damit ein intrinsischer Kondensator (z.B. das Sandwich leitenden Werkstoff, nichtleitenden (besonders dielektrisch ausgeprägter) Werkstoff, leitender Werkstoff bilden in Flussrichtung dieser Aufzählung einen Kondensator) implementiert werden.

Die erfindungsgemäße Spule in LTCC-Technik verwendet einen direkt in ein keramisches Substrat integrierten Aufbau. Dies wird durch eine sandwichartige Schichtung aus wechselweise übereinander gelegten offenen Metallringen und Keramiklagen erfolgen, wobei benachbarte Metallringe an ihren offenen Enden über Durchführungen elektrisch verbunden sind. Damit lassen sich kompakte Spulen mit ausreichenden Induktivitätswerten realisieren.

Auch eine Ausbildung der Spule als planare Spule auf einem flexiblen Substrat ist möglich. Dies hat den Vorteil, dass das Bauelement dann aufgrund der Flexibilität des Substrats gebogen und platzsparend in der elektronischen Baugruppe des Hochfrequenz-Filters untergebracht werden kann. Eine besonders kompakte Ausführungsform unter Verwendung eines flexiblen Substrats stellt die Ausbildung der Spule als Spirale dar, da die Leiterbahnen dann in einer Ebene liegen. Dieses Konzept kann auch in Form mehrerer Spiralen auf einem flexiblen Substrat umgesetzt werden. Letzteres kann dann vorzugsweise zu einem Zylinder gewickelt werden, sodass sich eine optimal an den Einbau in einer Elektrodenvorrichtung angepasste Konfiguration ergibt.

Weitere Merkmale, Einzelheiten und Vorteile der Erfindung ergeben sich aus der nachfolgenden Beschreibung, in der Ausführungsbeispiele der Erfindung näher erläutert sind. Es zeigen:
- Fig. 1: einen schematischen Längsschnitt einer Elektrodenvorrichtung in ihrem distalen Endbereich,
- Fig. 2: eine perspektivische Ansicht der Leiterbahnen einer LTCC-Spule,
- Fig. 3: eine Draufsicht auf eine als Spirale ausgebildete LTCC-Spule,
- Fig. 4A: eine Darstellung eines Spulenzylinders mit vier spiralig aufgebauten Spulen,
- Fig. 4B: eine Darstellung eines Spulenzylinders mit vier spiralig aufgebauten Spulen,
- Fig. 5A: eine Darstellung eines Querschnitts durch einen LTCC Kondensators, und
- Fig. 5B: eine Darstellung eines Querschnitts durch einen LTCC Kondensators, der mit Durchführungen verbunden ist.

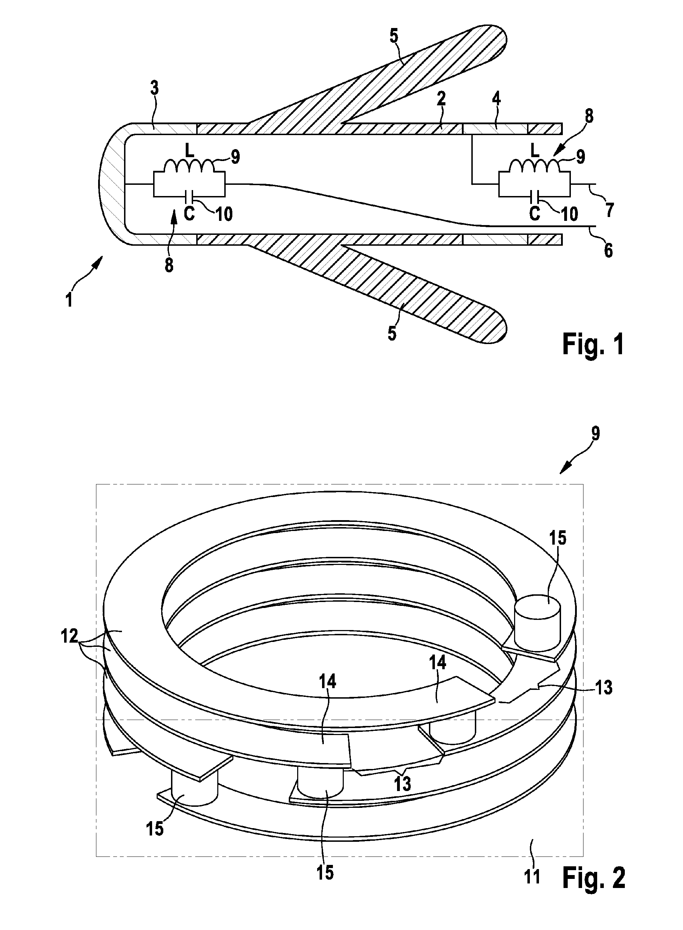

In der Fig. 1 ist der Bereich des distalen Endes 1 einer Elektrodenvorrichtung dargestellt, die einen langgestreckten Elektrodenkörper 2 aus einem flexiblen, isolierenden Schlauch aufweist.

An der Spitze des Elektrodenkörpers 2 ist ein üblicherweise als Tip-Elektrode bezeichneter Spitzen-Kontaktpol 3 befestigt, der beim Einsatz der Elektrodenvorrichtung in Kontakt beispielsweise mit Herzgewebe gebracht wird.

In proximaler Richtung vor dem Spitzen-Kontaktpol 3 ist mit Abstand dazu ein Ring-Kontaktpol 4 im Elektrodenkörper 2 befestigt, der ebenfalls zur Abgabe elektrokardiologischer Reizströme oder zur Messung elektrokardiologischer Signale dient. Zwischen Spitzen- und Ring-Kontaktpol 3, 4 stehen vom Elektrodenkörper 2 mehrere Silikon-Anker 5 zur Befestigung der Elektrodenvorrichtung an geeigneter Stelle in Herzgewebe schräg in proximaler Richtung ab.

Die elektrische Verbindung zwischen einem nicht näher dargestellten elektromedizinischen Implantat, wie beispielsweise einem Herzschrittmacher, und dem Spitzen- bzw. Ring-Kontaktpol 3, 4 wird jeweils über eine elektrische Zuleitung 6, 7 vorgenommen, die in aller Regel als Wendel ausgebildet sind.

Wie schematisch nach Art eines Blockschaltbildes in Fig. 1 angedeutet ist, sind in den beiden Zuleitungen 6, 7 jeweils ein Hochfrequenz-Filter 8 eingeschaltet, die aus einem LC-Schwingkreis bestehend aus einer Parallelschaltung einer Spule 9 und eines Kondensators 10 besteht.

Die die Spule 9 und den Kondensator 10 realisierenden elektronischen Bauelemente sind jeweils als Miniatur-Bauelemente in LTCC-Technik ausgelegt, wie sie bereits eingangs der Beschreibung ausführlich dargelegt wurde. Zur Vermeidung von Wiederholungen kann darauf verwiesen werden.

Die Integration des Kondensators 10 kann in ein herkömmliches SMD-Bauteil mit einer kleinen Bauform erfolgen. Baugrößen mit den Kennungen 0402, 0201 oder 1005 sind in diesem Zusammenhang vorteilhaft. Der Kondensator 10 ist dabei als LTCC-Bauteil in dielektrisches Material integriert. Eine Alternative ist die Montage eines LTCC-Kondensators 10 auf ein dielektrisches Material durch Löten, Schweißen oder Kleben mit leitfähigem Klebstoff.

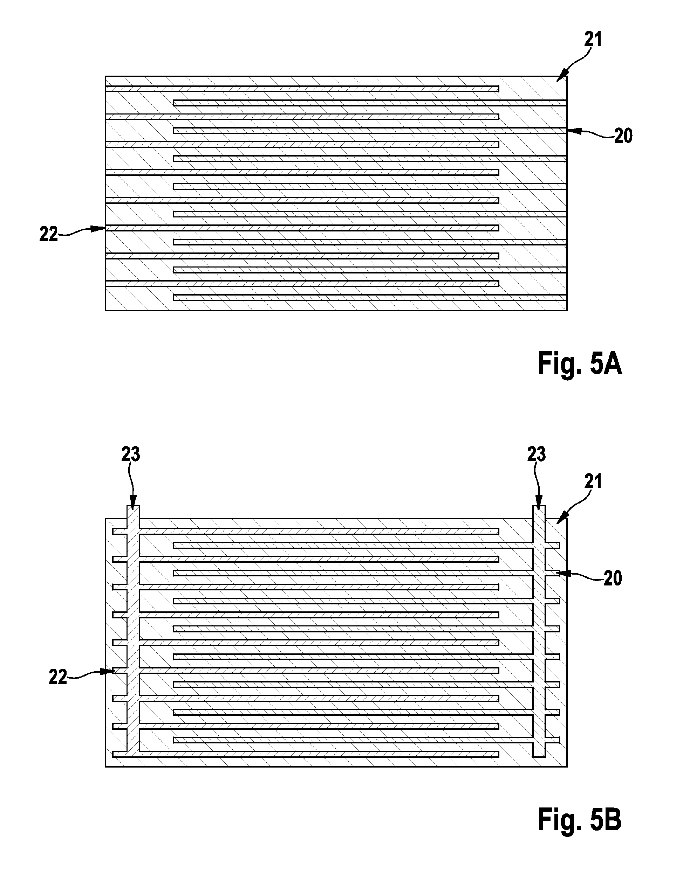

Bei der Nutzung eines dielektrischen Materials als keramisches Dielektrikum kann der Kondensator 10 als intrinsischer Kondensator implementiert werden. Damit kann das Volumen des Kondensators in der gesamten elektronischen Baugruppe des Hochfrequenz-Filters 8 verteilt werden, so zeigt beispielsweise die Fig. 5A den Querschnitt durch einen LTCC Kondensator. Die kammartigen Strukturen 20 und 22 stellen die Metallisierungsschichten innerhalb des LTCC 21 Substrates dar. Zwei benachbarte Metallschichten bilden mit dem dazwischen liegenden Keramiksubstrat je einen Kondensator.

Diese Einzelkondensatoren können, wie in Fig. 5B gezeigt, elektrisch so verschaltet werden, dass sie einen Kondensator mit größerer Kapazizät bilden. Die elektrischen Verbindungen 23 zwischen den einzelnen Schichten werden in der LTCC Technik üblicherweise als Durchführung umgesetzt. Folgende Abbildung zeigt eine mögliche Ausführungsform, bei der die Anschlüsse des Kondensators auf die Oberfläche geführt sind.

Im speziellen Anwendungsfall des Kondensators als Teil eines Filter können die Anschlüsse auch direkt mit der oder den Spulen innerhalb des Substrates verbunden werden.

Aus Fig. 2 wird der Aufbau einer Spule 9 in LTCC-Technik deutlich. Eine solche Spule 9 ist direkt in ein keramisches Substrat 11 (gestrichelt in Fig. 2 dargestellt) integriert, indem die Spule 9 sandwichartig aus wechselweise übereinander geschichteten, offenen Metallringen 12 und dazwischen - der Übersichtlichkeit halber weggelassenen - Keramiklagen aufgebaut ist. Die Metallringe 12 sind ringscheibenartig aus Flachmaterial gebildet und in Schichtungsrichtung überlappend parallel zueinander angeordnet. Der Spalt 13 zwischen den offenen Enden 14 der Metallringe 12 erstreckt sich über wenige Umfangsgrade der Metallringe 12. Die Spalte 13 von benachbarten Metallringen 12 sind jeweils in peripherer Richtung so zueinander versetzt, dass sie sich außer Überdeckung befinden.

Die elektrische Verbindung zwischen den einzelnen Metallringen 12 wird jeweils über Durchführungen 15 in Form von kurzen Leitungsstücken vor den jeweiligen Enden der Metallringe 12 hergestellt.

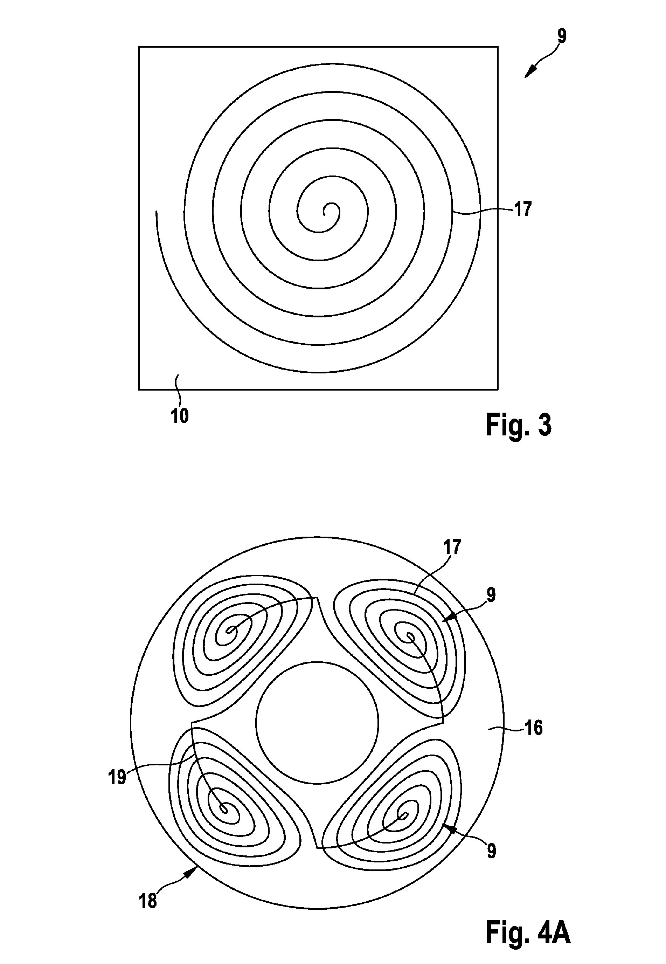

In Fig. 3 ist höchst schematisch eine alternative Ausführungsform der Spule 9 dargestellt. Diese ist als planare Spule auf einem flexiblen Substrat 16 ausgebildet, indem die Spulenleitungen 17 als Spirale auf das Substrat 16 gedruckt sind. Aufgrund seiner Flexibilität kann das Substrat 16 dann gebogen werden, sodass die gesamte Spule 9 platzsparend im entsprechenden elektronischen Bauelement integriert werden kann.

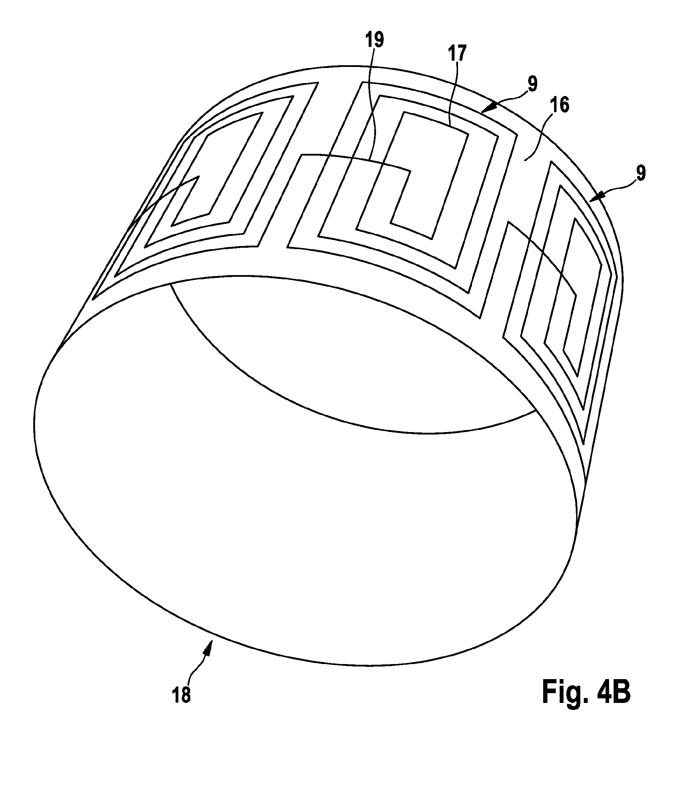

Die Fig. 4A und 4B schließlich zeigen die Integration von vier Spulen 9 in Form jeweils aufgedruckter Spiralen auf einem flexiblen Substrat 16. Die vier Spulen 9 sind dabei seriell verschaltet, wobei die Verbindung zwischen dem inneren Ende der Spulenleitung 17 und dem äußeren Anfang der jeweils nächsten Spule 9 durch eine gerade Verbindungsleitung 19 auf der Rückseite des flexiblen Substrates 16 erfolgen kann. In der Fig. 4 sind die vier Spulen 9 auf dem Substrat angedeutet, wobei Letzteres zu einem Spulenzylinder 18 gewickelt ist.

### Bezugszeichenliste

- 1: Distales Ende
- 2: Elektrodenkörper
- 3: Spitzen-Kontaktpol
- 4: Ring-Kontaktpol
- 5: Silikon-Anker
- 6: Zuleitung
- 7: Zuleitung
- 8: Hochfrequenz-Filter
- 9: Spule
- 10: Kondensator
- 11: Substrat
- 12: Metallringe
- 13: Spalt
- 14: Offenes Ende
- 15: Durchführung
- 16: Flexibles Substrat
- 17: Spulenleitung
- 18: Spulenzylinder
- 19: Verbindungsleitung
- 20: Metallisierungsschichten im LTCC
- 21: LTCC Substrat
- 22: Metallisierungsschichten im LTCC
- 23: Elektrische Verbindung

## Patentansprüche

1. Elektrodenvorrichtung für aktive medizinische Implantate umfassend einen langgestreckten Elektrodenkörper (2) mit einem proximalen und einem distalen Ende (1),
einen Spitzen-Kontaktpol (3) am distalen Ende (1) und/oder einen Ring-Kontaktpol (4) vor dem distalen Ende (1),
elektrische Zuleitungen (6, 7) zu dem Spitzen- bzw. Ring-Kontaktpol (3, 4), und
einen aus einem oder mehreren elektronischen Bauelementen (9, 10) bestehenden Hochfrequenz-Filter (8) in mindestens einer der Zuleitungen (6, 7), der dem Spitzen- bzw. Ring-Kontaktpol (3, 4) zugeordnet ist, wobei das oder die elektronischen Bauelemente (9, 10) jeweils als Miniatur-Bauelement in LTCC-Technik ausgeführt ist und die Spule (9) direkt in einem keramischen Substrat (11) integriert aufgebaut ist,
**dadurch gekennzeichnet, dass**
die Spule (9) sandwichartig aus wechselweise übereinander geschichteten offenen Metallringen (12) und Keramiklagen aufgebaut ist, wobei benachbarte Metallringe (12) an ihren offenen Enden (14) über Durchführungen (15) elektrisch verbunden sind.

2. Elektrodenvorrichtung nach Anspruch 1, **dadurch gekennzeichnet, dass** der Hochfrequenz-Filter (8) einen Kondensator (10) und eine Spule (9) in LTCC-Technik aufweist.

3. Elektrodenvorrichtung nach Anspruch 1 oder 2, **dadurch gekennzeichnet, dass** die Spule (9) als planare Spule auf einem flexiblen Substrat (16) ausgebildet ist.

4. Elektrodenvorrichtung nach Anspruch 3, **dadurch gekennzeichnet, dass** die Spule (9) als Spirale auf dem flexiblen Substrat (16) ausgebildet ist.

5. Elektrodenvorrichtung nach Anspruch 4, **dadurch gekennzeichnet, dass** mehrere Spiralen auf einem flexiblen Substrat (16) aufgebracht sind.

6. Elektrodenvorrichtung nach Anspruch 5, **dadurch gekennzeichnet, dass** das flexible Substrat (16) zu einem Zylinder (18) gewickelt ist.

## Claims

1. An electrode device for active medical implants, comprising
an elongate electrode body (2) having a proximal and a distal end (1),
a tip contact pole (3) at the distal end (1) and/or a ring contact pole (4) before the distal end (1),
electrical supply leads (6, 7) to the tip and/or ring contact pole (3, 4), and
a high-frequency filter (8), consisting of one or more electronic components (9, 10), in at least one of the supply leads (6, 7), which high-frequency filter is associated with the tip and/or ring contact pole (3, 4), wherein the electronic component or the electronic components (9, 10) is/are embodied in each case as a miniature component formed by LTCC technology and the coil (9) is directly integrated in a ceramic substrate (11),
**characterised in that**
the coil (9) is constructed in a sandwich-like manner from open metal rings (12) and ceramic layers layered above one another alternately, wherein adjacent metal rings (12) at the open ends (14) thereof are electrically connected via passages (15).

2. The electrode device according to Claim 1, **characterised in that** the high-frequency filter (8) has a capacitor (10) and a coil (9) formed by LTCC technology.

3. The electrode device according to Claim 1 or 2, **characterised in that** the coil (9) is formed as a planar coil on a flexible substrate (16).

4. The electrode device according to Claim 3, **characterised in that** the coil (9) is formed as a spiral on the flexible substrate (16).

5. The electrode device according to Claim 4, **characterised in that** a number of spirals are applied to a flexible substrate (16).

6. The electrode device according to Claim 5, **characterised in that** the flexible substrate (16) is rolled to form a cylinder (18).

## Revendications

1. Dispositif d'électrode pour des implants médicaux actifs, comprenant un corps d'électrode allongé (1) avec une extrémité proximale et une extrémité distale (1),
un pôle de contact en pointe (3) à l'extrémité distale (1) et/ou un pôle de contact annulaire (4) avant l'extrémité distale (1),
des lignes d'alimentation électriques (6, 7) vers le pôle de contact en pointe ou annulaire (3, 4), et
un filtre haute fréquence (8) constitué d'un ou de plusieurs composants électroniques (9, 10) dans au moins l'une des lignes d'alimentation (6, 7), lequel est attribué au pôle de contact en pointe ou annulaire (3, 4), dans lequel le ou les composants électroniques (9, 10) sont respectivement conçus comme un composant miniature avec la technique LTCC, et la bobine (9) est intégrée directement dans un substrat céramique (11),
**caractérisé en ce que**
la bobine (9) est conçue comme un sandwich, à partir d'anneaux métalliques ouverts (12) et de couches de céramique, superposés en alternance, des anneaux métalliques (12) voisins étant électriquement reliés à leurs extrémités ouvertes (14) par le biais de passages (15).

2. Dispositif d'électrode selon la revendication 1, **caractérisé en ce que** le filtre haute fréquence (8) comporte un condensateur (10) et une bobine (9) selon la technique LTCC.

3. Dispositif d'électrode selon la revendication 1 ou 2, **caractérisé en ce que** la bobine (9) est conçue comme une bobine planaire sur un substrat souple (16).

4. Dispositif d'électrode selon la revendication 3, **caractérisé en ce que** la bobine (9) est conçue comme une spirale sur le substrat souple (16).

5. Dispositif d'électrode selon la revendication 4, **caractérisé en ce que** plusieurs spirales sont appliquées sur un substrat souple (16).

6. Dispositif d'électrode selon la revendication 5, **caractérisé en ce que** le substrat souple (16) est enroulé en un cylindre (18).
